# EUROPEAN PATENT SPECIFICATION

(11) **EP 3 441 062 B1**
(45) Date of publication and mention of the grant of the patent: **29.11.2023**
(21) Application number: 18187679.8
(22) Date of filing: 07.08.2018
(51) Int. Cl.: A61K 9/00, A61K 9/107, A61K 9/51, A23L 33/00, A61K 36/79, A61K 31/122, A61K 31/714, A61K 31/592, A61K 33/04, A61K 33/06, A61K 33/26, A61K 33/32, A61K 36/41, A23L 33/105, A23L 33/135, A23L 33/155, A61K 33/30, A61K 31/4415, A61K 31/375

(54) **SEAWATER AND SALT POWDER BALANCING FORMULATIONS AND NUTRITIONAL AND PHARMACEUTICAL COMPOSITIONS**
MEERWASSER UND SALZPULVERAUSGLEICHSFORMULIERUNGEN UND PHARMAZEUTISCHE UND NAHRUNGSZUSAMMENSETZUNGEN
FORMULATIONS D'ÉQUILIBRAGE D'EAU DE MER ET DE POUDRE DE SEL ET COMPOSITIONS NUTRITIONNELLES ET PHARMACEUTIQUES

(30) Priority: 07.08.2017 EP 17185171
(43) Date of publication of application: 13.02.2019
(73) Proprietor: Corbinos Pubill, Maria Teresa, 08022 Barcelona (ES); Salama, Magdy, 08022 Barcelona (ES)
(72) Inventor: Zoser B., Salama, 88214 Ravensburg (DE)
(74) Representative: Hertin und Partner Rechts- und Patentanwälte PartG mbB

(56) References cited:
- EP-A1- 2 759 290
- WO-A1-2015/082356
- WO-A1-2017/009870
- US-A1- 2009 061 050
- MA'OR Z ET AL: "Skin smoothing effects of dead sea minerals: comparative profilometric evaluation of skin surface", INTERNATIONAL JOURNAL OF COSMETIC SCIENCE, KLUWER ACADEMIC PUBLISHERS, DORDRECHT, NL, vol. 19, 1 January 1997 (1997-01-01), pages 105-110, XP002257163, ISSN: 0142-5463, DOI: 10.1046/J.1467-2494.1997.171705.X

## Description

The invention relates to a composition, comprising seawater and an oil-soluble vitamin, and one or more minerals, trace elements, vitamins, prebiotics, probiotics, enzymes, co-enzymes, parts of a plant and/or plant extracts, preferably parts of a fruit or vegetable or fruit or vegetable extracts, and one or more oils, wherein said composition is obtained using a particle method, comprising homogenizing, and/or sonicating a seawater and oil composition, leading to liposomes or vesicles of nanoparticle size, or mixtures thereof.

The invention further relates to methods for producing said compounds, agents and compositions. The present invention relates to healthcare products. In particular the present invention relates to a composition that is useful in preventing or treating health conditions using the different formulations for oral use.

The present invention is based on the surprising and unexpected development and findings that the compositions comprising of nanoparticles of seawater/salt, olive oil, ozonated olive oil, oil-soluble vitamins, plants, fruits, and/or vegetables can prevent, attenuate and/or compensate the symptoms of said minerals, trace elements, vitamins, and/or enzymes deficiencies.

### SUMMARY OF THE INVENTION

In the light of the prior art the technical problem underlying the present invention is to provide alternative or improved compositions which demonstrate prevention, attenuation, compensation, balancing, treatment of deficiencies of minerals, trace elements, vitamins, enzymes in the body of human and animals.

If prolonged, this depletion in minerals can cause severe deficiencies in the body functions affecting metabolism and cell growth and repair, further leading to major skin, cardiovascular, joint, muscle or nerve disorders.

A proposed solution to this problem consists in an intake of dietary supplements formulated to contain several different chemical elements (as compounds), a combination of vitamins and/or other chemical compounds, or a single element (as a compound or mixture of compounds), such as calcium (such as carbonate, citrate) or magnesium (as oxide, for example), chromium (usually as picolinate) or iron (as bis-glycinate).

However, in order to be absorbed by the body in an efficient and effective manner each mineral, vitamin and enzyme requires a co-factor or sometimes referred to as an ion pairing. Vitamin, mineral, protein and enzyme interaction and interdependency is well documented scientifically and recognized. For example, magnesium competes with fluoride and will decrease absorption of that mineral when taken at the same time.

Therefore, there are problems in providing an adequate intake of essential minerals which consists in formulating a balanced mineral composition that allows a good and effective absorption of the minerals in the body and their interaction with vitamins and enzymes.

There is a need for providing a balanced composition of minerals for cosmetic or pharmaceutical, nutraceutical and/or veterinary uses. Therefore, it is an aim of the present invention to provide a such composition of minerals for cosmetic, pharmaceutical nutraceutical and/or veterinary uses according to this invention.

The invention therefore relates to a composition as a dietary and nutritional supplement in an oral administration form, comprising:
(1) seawater, and
(2) one or more minerals, trace elements, vitamins, prebiotics, probiotics, enzymes, co-enzymes, parts of a plant and/or plant extracts, comprising at least one oil-soluble vitamin, and
(3) one or more oils.
   wherein said composition is obtained using a nanoparticle method, said method comprising homogenizing and/or sonicating a seawater and oil mixture, such that the composition comprises liposomes or vesicles of nanoparticle size, or mixtures thereof.

The particle method comprises any method that leads to nanoparticles forming in the composition, preferably by any kind of homogenizing, and/or sonicating, preferably methods leading to liposomes, vesicles, and/or nanodroplets formed by the oil. The particles may in some embodiments be vesicles, liposomes, nanodroplets, arising preferably from homogenizing the mixture comprising oil and other components. In some embodiments, the liposomes, vesicles, and/or nanodroplets may be of nanoparticle size. The particle method may therefore be characterized as a method comprising homogenizing, and/or sonicating a seawater and oil composition, leading preferably to liposomes, vesicles, and/or nanodroplets of nanoparticle size, or mixtures thereof.

In one embodiment of the invention, the particle method for the preparation of the composition comprises:
a. Mixing one or more items of component (2) as above, comprising an oil-soluble vitamin, with one or more oil(s);
b. Adding the oil mixture from step a. gradually to seawater, optionally mixed with additional items of component (2) as above, thereby mixing the oil mixture from step a. with seawater, optionally mixed with additional items of component (2) according to claim 1, wherein the gradual mixing occurs preferably by adding 10-35% of the oil mixture (or preferably 15, 20, 25, 30 or 33% of the oil mixture) from step a. every 10-15 minutes (or preferably 11, 12, 13, 14 minutes) over a period of 30 to 150 minutes (preferably approximately 45 to 75 minutes, more preferably approximately 60 minutes);
c. Bringing the temperature of the mixture in step b. to 40 to 60 degrees Celsius, preferably approximately 50 degrees Celsius; either during and/or after the mixing in step b.,
d. Vortexing, agitating, homogenizing, and/or sonicating the mixture in step b., either during and/or after the mixing in step b.; and
e. Formulating the mixture after step d. to a composition in the form of a lyophilized powder, tablet, capsule, dragée, sublingual tablet, granule, emulsion suitable for oral administration.

The invention also relates to a method of manufacturing the composition, as described herein. In some embodiments, the step e. is not comprised in the method, or used in the method to define the composition, but is carried out optionally or at a later time.

In some embodiments, the relative ratio of volume of the oil mixture from step a. to the seawater (optionally mixed with additional items of component (2) as above) is from 1:1000000 to 1000000:1, or 1:100000 to 100000:1, or 10000:1 to 1:10000, or 1000:1 to 1:1000, or 100:1 to 1:100, or 10:1 to 1:10, or approximately 1:1.

According to the invention, the composition comprises at least one oil-soluble vitamin, such as vitamin E, preferably present in the oil-mixture of step a.

In the embodiments below, the items recited are intended preferably in combination with the components:
(1) seawater, and
(2) one or more minerals, trace elements, vitamins, prebiotics, probiotics, enzymes, co-enzymes, parts of a plant and/or plant extracts, comprising at least one oil-soluble vitamin, and
(3) one or more oils.

In one embodiment of the invention, the composition comprises:
a. one or more minerals and/or trace elements; and
b. one or more vitamins, enzymes and/or co-enzymes.

In one embodiment of the invention, the composition comprises:
a. one or more minerals and/or trace elements;
b. one or more vitamins, enzymes and/or co-enzymes; and
c. one or more parts of a plant or plant extract, preferably parts of a fruit or vegetable or fruit or vegetable extract.

In one embodiment of the invention, the composition comprises:
a. one or more minerals and/or trace elements;
b. one or more vitamins, enzymes and/or co-enzymes;
c. one or more parts of a plant or plant extract, preferably parts of a fruit or vegetable or fruit or vegetable extract; and
d. one or more prebiotics and/or probiotics.

In one embodiment of the invention, the one or more minerals and/or trace elements are selected from iron, zinc, manganese, calcium, selenium and/or potassium.

In one embodiment of the invention, the one or more parts of a plant or plant extract are selected from maca, rhodiola and/or schisandra.

In one embodiment of the invention, the one or more parts of a plant or plant extract are selected from guarana, red yeast rice, green tea,
In one embodiment of the invention, the one or more vitamins, enzymes and/or co-enzymes are selected from co-enzyme Q10, vitamin C, D, A, B6, B12, D3, C, and/or vitamin E.

In one embodiment of the invention, the one or more parts of a plant or plant extract, preferably parts of a fruit or vegetable or fruit or vegetable extract, are selected from raspberries, strawberries, blackberries, blueberries, sour cherries, apricots, apples, aronia berries, red currants, plums, lemons, pears, kiwis, peaches, banana, and/or pineapple.

In one embodiment of the invention, the composition comprises particles in the form of vesicles comprising vitamin E, and olive oil, in addition to at least one mineral, trace element and/or vitamin.

In one embodiment of the invention, the composition comprises:
a. at least one of manganese, calcium, selenium, potassium,
b. at least one of maca, rhodiola, schisandra, probiotic,
c. at least one of co-enzyme Q10, vitamin C, D, and
d. at least one of raspberries, strawberries, blackberries, blue berries, sour cherries, apricots, apples, aronia berries, red currants, plums, lemons, pears, kiwis, peaches, banana, and/or pineapple.

In one embodiment of the invention, the composition comprises:
a. at least one of cardamom, anise, flaxseeds, probiotics,
b. at least one of Co-enzyme Q10, vitamin A, B6, B12, D3, C, E,
c. at least one of iron, zinc, selenium, manganese, and
d. at least one of raspberries, strawberries, blackberries, blueberries, sour cherries, apricots, apples, aronia berries, red currants, plums, lemons, pears, kiwis, peaches, banana, and/or pineapple.

In one embodiment of the invention, the composition comprises:
a. at least one of guarana, red yeast rice, green tea, probiotic,
b. at least one of co-enzyme Q10, vitamin C, B6, B12, D3,
c. at least one of zinc, selenium, calcium, manganese, and
d. at least one of raspberries, strawberries, blackberries, blueberries, sour cherries, apricots, apples, aronia berries, red currants, plums, lemons, pears, kiwis, peaches, banana, and/or pineapple.

The invention further relates to a method for manufacturing or producing a composition as described herein.

In one embodiment, the method comprises employing the following components:
(1) seawater,
(2) one or more minerals, trace elements, vitamins, prebiotics, probiotics, enzymes, co-enzymes, parts of a plant and/or plant extracts, comprising at least one oil-soluble vitamin, and
(3) one or more oils.

In one embodiment, the method comprises a nanoparticle method, comprising preferably
a. Mixing one or more items of component (2) as above, comprising an oil-soluble vitamin, with one or more oil(s);
b. Adding the oil mixture from step a. gradually to seawater, optionally mixed with additional items of component (2) as above, thereby mixing the oil mixture from step a. with seawater, optionally mixed with additional items of component (2) according to claim 1,
   wherein the gradual mixing occurs preferably by adding 10-35% of the oil mixture from step a. every 10-15 minutes over a period of 30 to 150 minutes, preferably approximately 45 to 75 minutes, more preferably approximately 60 minutes;
c. Bringing the temperature of the mixture in step b. to 40 to 60 degrees Celsius, preferably approximately 50 degrees Celsius; either during and/or after the mixing in step b.,
d. Vortexing, agitating, homogenizing, and/or sonicating the mixture in step b., either during and/or after the mixing in step b.; and
e. Formulating the mixture after step d. to a composition in the form of a lyophilized powder, tablet, capsule, dragée, sublingual tablet, granule, emulsion, suspension, or composition suitable for oral administration.

In further embodiments, the composition or method may be described as being characterized in that composition is prepared as a lyophilized powder, lyophilized mixture, dried mixture as powder, dried mixture, homogenized mixture, suspension, emulsion, effervescent granulates, effervescent tablet or sublingual tablets.

In one embodiment, the composition comprises selenium, selenium salts such as sodium selenite pentahydrate in seawater using seawater, seawater salts as powder, water for injection, or distilled water with or without other ingredients employing suitable preservatives, absorption promoters to enhance bioavailability, solubilizing or dispersing agents known in the art are prepared according to techniques well known in the art of preparation and formulations, not limited to for oral and/or sterile parenteral administration and/or transmucosal, transbuccal, topical, vaginal, inhalation, and/or for use in the eyes and nose.

In one embodiment, kits comprising the composition or items for manufacturing the composition are encompassed, as is packaging of the composition of the present application using containers not limited to ampoule, bag, blister, bottle, cartridge, filled syringes, gas cylinders, injected needles, injection syringes, single dose containers and any other state-of-the art known, and/or known in the art packaging materials, containers.

The dosing and the frequency of the application, intake, administration and use of the compositions according to this application will be determined and adjusted to meet the objective of their use, as according to the knowledge of a skilled person.

The invention further relates to a composition comprising seawater, morphous and/or amorphous seawater salt, seawater salt powder, with minerals, trace elements, vitamins, plants, plant extracts, probiotics, enzymes, co-enzymes, fruits, vegetables and/or oils, using a nanoparticle method.

In one embodiment, the nanoparticle method for the preparation of the composition comprises:
- Mixing vitamin E and/or oil(s),
- Addition of the oil mixture gradually in small quantities over a period of 1 hour to the seawater and the ingredients such as minerals, trace elements, vitamins, plants, plant extracts, probiotics, enzymes, co-enzymes, fruits, and/or vegetables,
- Bring the temperature of the mixture to 50° Celsius,
- Vortex, agitate, homogenize, and/or sonicate,
- Formulate the composition with the inactive ingredients to lyophilized powder, tablets, capsules, dragées, sublingual tablets, granules, as single and/or multiple dose.

In one embodiment, the composition comprises of vesicles and non-vesicles of vitamin E and/or olive oil with seawater, minerals, trace elements and/or vitamins.

In one embodiment, the composition comprises of vesicle of Seawater, Manganese, Calcium, Selenium, Potassium, Maca, Rhodiola, Schisandra, Probiotic, Co-enzyme Q10, Vitamin C, and/or Vitamin D.

In one embodiment, the composition comprises of seawater, cardamom, anis, flaxseeds, probiotic,co-enzyme Q10, iron, zinc, selenium, manganese, vitamin A, B6, B12, D3, C, and/or vitamin E.

In one embodiment, the composition comprises of seawater, guarana, red yeast rice, green tea, probiotic, co-enzyme Q10, zinc, selenium, calcium, manganese, vitamin C, B6, and/or vitamin B12
In one embodiment, the composition comprises of seawater, raspberries, strawberries, blackberries, blueberries, sour cherries,apricots, apples, aronia berries,redcurrants, plums, lemons, pears, kiwis, peaches, banana, and/or pineapple.

In one embodiment, the composition comprises of Seawater, manganese, calcium, selenium, potassium, maca, rhodiola, schisandra, probiotic, co-enzyme Q10, vitamin C, D, raspberries, strawberries, blackberries, blue berries, sour cherries, apricots, apples, aronia berries,red currants, plums, lemons, pears, kiwis, peaches, banana, and/or pineapple.

In one embodiment, the composition comprises of seawater, cardamom, anise, flaxseeds, probiotics, Co-enzyme Q10, iron, zinc, selenium, manganese, vitamin A, B6, B12, D3, C, E, plus, raspberries, strawberries, blackberries, blueberries, sour cherries,apricots, apples, aronia berries, redcurrants, plums, lemons, pears, kiwis, peaches, banana, and/or pineapple.

In one embodiment, the composition comprises of seawater, guarana, red yeast rice, green tea, probiotic, co-enzyme Q10, zinc, selenium, calcium, manganese, vitamin C, B6, B12, D3, plus, raspberries, strawberries, blackberries, blueberries, sour cherries, apricots, apples, aronia berries, redcurrants, plums, lemons, pears, kiwis, peaches, banana, and/or pineapple.

In one embodiment, the composition is characterized in that the composition is prepared as water solution, lyophilized powder, lyophilized mixture, dried mixture as powder, dried mixture, homogenized mixture, suspension, emulsion, effervescent granulates, effervescent tablet, sublingual tablets, transmucosal formulation, and/or parenteral preparation.

In one embodiment, the composition comprises selenium, selenium salts such as sodium selenite pentahydrate in seawater using seawater, seawater salts as powder, water for injection, or distilled water with or without other ingredients employing suitable preservatives, absorption promoters to enhance bioavailability, solubilizing or dispersing agents known in the art are prepared according to techniques well known in the art of preparation and formulations, not limited to for oral and/or sterile parenteral administration and/or transmucosal, transbuccal, topical, vaginal, inhalation, and/or for use in the eyes and nose.

Further embodiments and features of the invention relate to:
1. Compositions comprising sea water with minerals, trace elements, vitamins, comprising at least one oil-soluble vitamin, fruits vegetables and oils, preferably sea water, sea water salt powder, and to use said compositions for the support of the body balance and/or compensation of the deficiencies of the body on minerals, trace elements, vitamins and/or enzymes.
2. Compositions comprising of vesicle of vitamin E and olive oil with sea water, minerals, trace elements and vitamins.
3. Composition comprising of sea water, manganese, calcium, selenium, potassium, Vitamin C, Vitamin D with or without olive oil and lemon.
4. Compositions comprising of sea water, iron, zinc, selenium, manganese, Vitamin A, Vitamin B6, Vitamin B12, Vitamin C, Vitamin E with or without olive oil and lemon.
5. Compositions comprising of sea water, zinc, selenium, calcium, manganese, Vitamin C, Vitamin B6, Vitamin B12.
6. Compositions comprising of sea water, fruits, raspberries, strawberries, blackberries, blueberries, sour Cherries, apricots, apples, aronia Berries, redcurrants, blums.
7. Compositions comprising sea water, manganese, calcium, selenium, potassium, Vitamin C, Vitamin D with or without olive oil and lemon and fruits, i.e. raspberries, strawberries, blackberries, blueberries, sour Cherries, apricots, apples, aronia Berries, redcurrants, plums.
8. Compositions comprising sea water, iron, zinc, selenium, manganese, Vitamin A, Vitamin B6, Vitamin B12, Vitamin C, Vitamin E with or without olive and fruits, i.e. raspberries, strawberries, blackberries, blueberries, sour Cherries, apricots, apples, aronia Berries, redcurrants, plums.
9. Compositions comprising of sea water, zinc, selenium, calcium, manganese, Vitamin C, Vitamin B6, Vitamin B12 and fruits, i.e. raspberries, strawberries, blackberries, blueberries, sour Cherries, apricots, apples, aronia Berries, redcurrants, plums.
10.Compositions comprising sea water, manganese, calcium, selenium, potassium, Vitamin C, Vitamin and Vegetable all parts.
11.Compositions suitable for the treatment of the skin comprising an artificial or natural sea water together with additional magnesium, minerals, trace elements and to use said compositions for the skin health selected from the group comprising acne, eczema, rosacea, seborhhea and psoriasis.
12.According to any one of the preceding embodiments 1-10 or other embodiments as disclosed herein, characterized in that the composition is prepared as water solution.
13.According to any one of the preceding embodiments 1-10 or other embodiments as disclosed herein, characterized in that the composition is prepared as lyophilysed powder.
14.According to any one of the preceding embodiments 1-10 or other embodiments as disclosed herein, characterized in that the composition is prepared as lyophilysed mixture.
15.According to any one of the preceding embodiments 1-10 or other embodiments as disclosed herein, characterized in that the composition is prepared as dried mixture as powder.
16.According to any one of the preceding embodiments 1-10 or other embodiments as disclosed herein, characterized in that the composition is prepared as dried mixture.
17.According to any one of the preceding embodiments 1-10 or other embodiments as disclosed herein, characterized in that the composition is prepared as homogenized mixture.
18.According to any one of the preceding embodiments 1-10 or other embodiments as disclosed herein, characterized in that the composition is prepared as suspension.
19.According to any one of the preceding embodiments 1-10 or other embodiments as disclosed herein, characterized in that the composition is prepared as emulsion.
20.According to any one of the preceding embodiments 1-10 or other embodiments as disclosed herein, characterized in that the composition is prepared with sea water and/or sea water salt.
21.According to any one of the preceding embodiments 1-10 or other embodiments as disclosed herein, characterized in that the composition is prepared as effervescent granulates.
22.According to any one of the preceding embodiments 1-10 or other embodiments as disclosed herein, characterized in that the composition is prepared as effervescent tablet.
23.According to any one of the preceding embodiments 1-10 or other embodiments as disclosed herein, characterized in that the composition is prepared for oral use.
24.According to any one of the preceding embodiments 1-10 or other embodiments as disclosed herein, characterized in that the composition is prepared for use in bottles, cap of bottles, cups, cap of cups, containers, bags, sachets, containers.
25. Method for the preparation of the compositions according to embodiments 1-10 or other embodiments as disclosed herein, comprising
   - Mix Vitamin E and the oil(s).
   - Add the oil mixture gradually in small quantities, over a period of one hour to the sea water prepared from sea water powder and trink water or mineral water.
   - The mixture will be brought to a temperature of 50C° under agitation.
   - The mixture will be homogenized (high speed homogenization or high pressure homogenization) and/or sonicated. Record all conditions e.g. temperature, time for any process such as vortex, agitation, homogenization, sonication etc.
   - High speed homogenization, high pressure homogenization and/or sonication could be used for the preparation of the nanoparticles of the in oil encapsulated vitamin E and the sea water.
   - Add this emulsion to the paste of ozonated olive gradually.
   - The mixture will be homogenized.
   - The formulation will be used for oral application.
26.Compositions of the present patent according to any one of the preceding claims comprising of selenium, selenium salts such as sodium selenite pentahydrate in sea water using sea water, sea water salts as powder.
27.Packaging of the compositions of the present patent according to embodiment 26 are containers for the packaging not limited to bottle, single dose containers and any other state-of-the art known, and/or known in the art packaging materials, containers.
28.The dosing and the frequency of the application, intake, administration and use of the compositions according to this patent will be determined and adjusted to meet the objective of their use.

The current formulation of vitamin, trace elements, enzymes, as presently prepared in the prior art, have a number of disadvantages such as:
- Will have high concentrations within a short amount of time, this means with a short Tmax (excessive dose)
- Vitamins are classified as either fat soluble (Vitamins A, D, E and K) or water soluble (vitamins B and C). This difference between the two groups is critical for the preparation of the multivitamins, enzyme, co-enzyme, plant extracts, probiotics, prebiotics, and/or mineral formulations with other ingredients of the compositions of this invention.
- The method according to the invention has provided surprisingly unexpected findings on the preparation of homogenous immediate and sustained release formulations of the claimed compositions.
- The current offering of products (e.g. vitamins, enzyme, co-enzyme, plant extracts, probiotics, prebiotics, and/or minerals) in the market place is such that the consumer needs to purchase several products in order to cover his/her needs, resulting in the following disadvantages:
   ∘ Negative impact on the quality of life of the consumer, who is exposed to several interactions with different products throughout the day
   ∘ Higher cost of the overall treatment
   ∘ High impact on the environment due to the usage of packaging and other elements of the product
   ∘ and a negative impact on the compliance of the consumer

The method and composition of the present invention has solved the above-mentioned disadvantages, showing the following unexpected and surprising findings:
1. Reproducible manufacturing procedure.
2. Suitability for use for the different ingredients of the composition according to the invention, not limited to seawater, salts, minerals, trace elements, vitamins, enzyme, co-enzyme, and/or probiotics.
3. Protection of the enzymes, co-enzymes, vitamins, probiotics and prebiotics from the degradation in blood.
4. Controlled immediate and sustained release delivery of the ingredients of the composition according to the invention.
5. Preparation of innovative formulations for the improvement of the bioavailability of the ingredients of the composition such as trans buccal route of administration.
6. Reproducible particle size and particle size distribution of the ingredients of the composition following the reconstitution of the lyophilized powder of the composition according to the invention.
7. Controlled delivery and release/targeting of the minerals and trace elements of the seawater.
8. Preparation of the homogenous mixture comprising of the different ingredients with complex structures such as fruits, vegetables, vitamins, enzymes, co-enzyme, and/or probiotics of the composition according to the invention.

It is also an object of the present invention to provide a composition with improved oral transmucosal absorption of minerals, trace elements, enzymes, coenzymes, plant extracts, and/or vitamins.

The present invention therefore relates to a composition comprising seawater, manganese, calcium, selenium, potassium, plant extracts, probiotics, coenzyme Q10, vitamin C, vitamin D with or without olive oil and the formation of vesicles with encapsulated minerals, trace elements and/or vitamins as source of sustainable energy, regulating the levels of insulin in blood.

The present invention therefore relates further to a composition comprising and containing effective amounts of seawater, iron, zinc, selenium, manganese, plant extracts, probiotics, coenzyme Q10, vitamins A, B6, B12, C, D3, with or without olive oil to support the elimination of toxic agents in the body.

The invention further provides compositions comprising and containing effective amounts of seawater, zinc, selenium, calcium, manganese, plant extracts, probiotics, coenzyme Q10, vitamin C, vitamin B6, vitamin B12, D3, with or without olive oil to mitigate the effects of cellular aging and supporting the improvement of quality of life.

Other preferred composition, which may be used according to the invention is comprising of fruits as lyophilized powder with seawater.

Other preferred composition, which may be used according to the invention is comprising of seawater, vegetables as lyophilized powder for reconstitution with water and/or mineral water.

The invention further provides compositions comprising and containing effective amounts of seawater, minerals, trace elements, plant extracts, probiotics, coenzyme Q10, vitamins, fruits, and/or vegetables as lyophilize powder for reconstitution with water and/or mineral water.

More particularly the invention relates to oral applications.

The composition according to the invention is active in preventing chronic diseases, improving health, delaying the aging process, increasing a person's disease-free years and promoting an aging well paradigm.

The features described herein, with respect to different embodiments, or various aspects of the invention, e.g. the composition and methods, are to be considered disclosed in combination with each other, e.g. the composition features may be used to described the method, and vice versa.

### BACKGROUND OF THE INVENTION

Minerals are important nutrients found in foods. Alone, they are inactive chemical elements, whether in a rock as calcium or in a cast iron pan. But in the body, they become operational, either structurally as bone, for example, or functionally as an electrolyte or hormone. Minerals are the most permanent part of a living organism, responsible for muscle responses, the transmission of messages through the nervous system, the maintenance of pH and the metabolism of food. Because the body is unable to manufacture them, minerals must come from the diet.

In other words, minerals are more important than vitamins, in that they cannot be synthesized by living matter. Thus, they are the spark-plugs in the chemistry of life, on which the exchanges of energy in the combustion of foods and the building of living tissues depend.

There are two groups of minerals in the body, major minerals and trace minerals. The former are required in amounts of 100 milligrams a day or more; the latter in lesser amounts. Different charts from different sources may disagree about some of them, but calcium, phosphorus, magnesium, sodium, chloride and potassium are generally listed as major, while iron, iodine, selenium, zinc, chromium, copper and manganese are often named as trace minerals. This group may also include molybdenum, silicon, boron, cobalt, sulfur and a few others. Each of these, both major and trace, has a specific job to do, and none is less important than any other.

In the body, some of these work as a team. Sodium and potassium work together in muscle contractions and relaxation. Calcium and magnesium have a similar working relationship, but each also does something else. The balance of these teams of minerals will be addressed separately later on. What's important to realize is that mineral depletion of the soil affects food value and ultimately, our health. Even though minerals move up the food chain from plant to animal to humans, deficiency is more common than imagined. Failure to rotate crops, breeding high-yield nutritionally-shallow cultivars, harvesting prematurely to prevent decay in transit, use of biocides, poor storage and handling, and careless cooking practices combine to denigrate food quality

If unbalanced beyond the body's ability to regulate them, minerals can accumulate and cause harm. It's not a good idea to take minerals merely. For example, calcium is needed for bones and a strong heartbeat. Even with perfect foods, diet supplies less calcium than what we need, but overload can precipitate as kidney stones and cause constipation.

There are 103 known minerals, at least 18 of these are necessary for good health. Mineral imbalance is epidemic. Osteoporosis is on the rise in our nation, 30 million people in the US over 50 are susceptible to fractures caused by mineral deficiencies in their bones. Over 1/3 of the women in America will have diagnosable osteoporosis in their lifetime. Osteoporosis isn't even diagnosed until you've lost 30% of your bone mass. Nearly 100% of Americans have some type of joint degeneration by the time they are 40 years old. Zinc deficiency is very common, evidenced by such health issues as; prostate cancer, breast cancer, hormonal imbalances, hydrochloric acid deficiency, skin cancer. Magnesium is a key element to keeping the cells metabolizing, involved in at least 300 functions in the body. It is particularly sensitive to stress, and can easily be lost. Most everyone is deficient in magnesium anymore, along will all of the main minerals of the body. It can take several years to re-mineralize the body. It can take 12 months to replace one mineral such as iron, so to replace more can take a while.

Chloride helps to make stomach acid and to maintain pH. Deficiency is almost unheard of. Magnesium deficit, however, is real, especially in children who avoid vegetables and because of mindless cooking techniques. Magnesium is a muscle relaxant and a part of more than three hundred enzymes, although most is in bones and teeth.

Phosphorus is found in every cell of the body, second in amount only to calcium. It is part of DNA, contributes to bone and teeth structure, and catalyzes the B-vitamins. Deficiency is unlikely, but overabundance, such as might come from too many cans of soda, can create calcium imbalance.

Potassium is an essential electrolyte. Living inside the cell, it controls water and acid-base balance while helping to relax a muscle contraction and to regulate heartbeat. Deficiency is common among the elderly, who are more apt to suffer chronic disease and to take medications that deplete this mineral.

Sodium maintains fluid balance and plays a role in muscle contraction, opposite potassium. Diarrhea, vomiting, heavy sweating and chronic illness can compromise sodium stores, but excess is more common.

Chromium enhances insulin function.

Copper helps to make collagen and hemoglobin.

Iodine keeps the thyroid healthy and helps to regulate energy production.

Iron helps to manufacture hemoglobin and may even benefit the immune system.

Manganese supports enzyme reactions and the overall health of the nervous system.

Molybdenum helps the body to use iron and to burn carbohydrates.

Sulfur, found in all cells and tissues, is needed to make collagen and for the synthesis of proteins.

Zinc is one of the more active minerals, involved in more than 200 enzymatic reactions. It is essential for growth and development, the regulation of insulin, immune function, prostate health and cell membrane integrity. Imbalance can interfere with copper function.

Minerals are also necessary in the production of hormones. For example, manganese and copper are necessary for catecholamine synthesis; zinc is involved in the production, storage, and secretion of insulin and is also necessary for growth hormones.

Another important function of the essential minerals is their necessity for enzyme activity and their content in RNA. Minerals are involved with enzymes in two ways:
1. They make up a part of, or are contained within, the enzyme (metalloenzyme).
2. They act as enzyme activators.

### Enzymes that require Zinc

- Carbotlic Anhydrase
- Carboxypeptidase
- Lactic Dehydrogenase
- Alkaline Phosphatase
- Alcohol Dehydrogenase
- Plasma Rnase

### Enzymes that require Copper

- Cytochrome C Oxidase
- Dopamine Hydroxylase
- Super Oxide Dismutase
- Uricase
- Tyrosinase
- Ascorbic Acid Oxidase

### Enzymes that require Iron

- Cytochrome C Oxidase
- Cytochrome C Reductase
- Peroxidase
- Xanthine Oxidase
- Aconitase
- NADH Dehydrogenase

**What can cause a mineral imbalance?**

Many factors can contribute to mineral imbalances. As Dr. Ashmead stated in his book, "Chelated Mineral Nutrition in Plants, Animals, and Man", *"There are at least eighteen barriers to mineral absorption, which means that the minerals we consume do not necessarily wind up in our bodies."*

### Diet

A major factor in contributing to a mineral imbalance is improper eating habits. Excessive intake of refined carbohydrates, alcohol, and fad diets can all lead to poor mineral nutrition. Even the mineral content of an unhealthy diet can be inadequate, depending upon the soil in which the food was grown or the method in which it was prepared.

### Stress

Stress, either physical or emotional, can lead to mineral imbalances. Certain nutrients such as the mineral zinc and the B-complex vitamins are lost in greater quantities due to increased stress. Nutrient absorption can also decrease when the body is under stress.

### Medications

Medications can deplete the body store of nutrient minerals or increase the levels of toxic metals. The well-known effects of diuretics include not only sodium loss but, in many cases, a potassium and magnesium loss. Antacids, aspirin, and oral contraceptive agents can lead to vitamin and mineral deficiencies as well as toxic metal excesses.

### Pollution

Toxic metals such as lead, mercury, and cadmium can interfere with mineral absorption and increase mineral excretion. From adolescence to adulthood the average person is continually exposed to a variety of toxic metal sources such as cigarette smoke (cadmium), copper and aluminum cookware, hair dyes (lead), lead based cosmetics, hydrogenated oils (nickel), antiperspirants (aluminum), and dental amalgams (mercury and cadmium). These are just a few of the hundreds of sources an individual, may be exposed to every day.

### Nutritional Supplements

Vitamin and mineral supplements can also lead to mineral imbalances. Calcium absorption is decreased in the presence of phosphorus. Vitamin C is required for iron absorption, but in excess amounts it can cause a copper deficiency. Vitamin D enhances calcium absorption but, in excess amounts can produce a magnesium deficiency, etc.

### Common health conditions associated with mineral imbalances

Continuing research is showing that many health conditions can be contributed to, or be aggravated by, various mineral imbalances and toxic metal excesses.

### Arteriosclerosis

Excessive calcium deposition on the arteries is higher in areas of the world with magnesium deficient soils and water. Magnesium is required in sufficient amounts to maintain a resistance to excessive calcium in the soft tissues of the body. An abnormal calcium to magnesium relationship can be readily identified through tissue mineral testing. The abnormal relationship of these minerals is not always revealed by other diagnostic tests.

### Hypercholesterolemia

Excess serum cholesterol and increased incidence of heart disease have been related to a low copper to zinc ratio in the serum. High zinc to copper ratios found in the tissues should warrant further investigation.

### Hyperactivity

Research reveals a strong relationship between hyperactivity in children and toxic metals such as cadmium, lead, and mercury, as well as high levels of iron, manganese, and copper.

### Migraine Headaches

Excessive nutrient mineral accumulation as well as toxic metals such as cadmium, mercury, and lead can contribute to migraine headaches.

### Learning Disabilities

Toxic metals such as lead have been implicated in learning disabilities and attention deficit disorders. Deficiencies of certain minerals have been shown to be associated with decreased academic performance.

Unfortunately, our bodies cannot make the minerals we need; minerals must be ingested from the foods we eat and from drinking fluids. Moreover, minerals are from living things, which mean they come from living foods- plants and animals, not processed foods. With that, many may not be meeting the minimum requirements for minerals they need and may never even think about it unless a relevant disease state occurs.

The biggest problem with improper mineral intake is what our bodies do when we are not getting our minimum mineral requirements. Most notably for homeostasis, which is the body's natural regulatory environment, our bodies need to maintain a pH balance so we are not too acidic or too alkaline. Without a proper pH balance, our bodies cannot do vital enzymatic reactions. Because of this important need for minerals, when there is a lack of minerals, the body will start leaching the minerals out of the bones. This of course leads to or exacerbates osteopenia and osteoporosis, which according to the Centers for Disease Control and Prevention affects 44 millions Americans, with 50% of women to be affected by osteoporosis and osteopenia at some point. Unfortunately, another issue is that people are exceedingly more acidic because of our diets due to cheese, coffee, tea, tap water, fats, and processed foods, which are all acidic. With the pH imbalance stemming from diet as well as poor mineral intake, the body is constantly trying to get back into balance so that we can function properly. That is the reason that ensuring that we are getting the minerals we need is an important task to put on our to-do list.

Therefore one object of invention is to provide a composition comprising seawater and/or seawater salts, with macro-minerals, trace minerals, vitamins, plants, coenzymes, enzymes and/or oils such as olive oil and/or ozonated olive oil thereof for use as a nutrition supplement for balancing, prevention, reduction, attenuation, elimination and/ or treatment of their deficiencies characterized in that said composition comprises preferably oral intake of said composition to a subject in need of said use.

It is also an object of the invention to provide a composition wherein the ingredients are selected from the groups of fruits, vegetables, plants, vitamins, probiotics, enzymes and coenzymes.

In a preferred embodiment of the invention olive oil, ozonated olive oil, vitamin E, olive oil, ozonated olive oil, coconut oil, hemp oil, and/or jojoba oil Will be used for the preparation of the vehicles (nano - and macro-particles) with the encapsulated supplements for the enhancement of their oral transmucosal absorption by oral intake.

In a preferred embodiment of the invention the seawater could be prepared using solid seawater salts, seawater salts as effervescent tablet with or without supplements and /or ingredients and/or extracts and or mixtures of fruit, plants and vegetables.

In a preferred embodiment of the invention is the use of the composition for the treatment of deficiencies of minerals, trace elements, vitamins, enzymes in patients suffering from cancer, AIDS, elderly people, children, and/or mass starvation. The term" treatment" may relate to prophylaxis, prevention, stabilization, attenuation of nutrition deficiencies. Prophylaxis is a preferred embodiment of the composition.

In one embodiment the use of the composition according to the present invention is characterized by transbuccal/transmucosal intake /administration of the composition. The preferred buccal route of delivery of the minerals, trace elements and vitamins provides the direct access to the systemic circulation through the jugular vein by passing the first pass hepatic metabolism leading to high bioavailability.

In a preferred embodiment the composition for use as selenium and/or selenite salt in seawater, seawater salt for oral intake according to the present invention is characterized in that the selenite preferably as sodium selenite, sodium selenite pentahydrate. Salts of selenite preferably to an organic acid, preferably selected from an acetic, trifluoracetic, propionic, succinic, glycolic, stearic, lactic, citric, ascorbic, or amino acid salt.

The invention further provides the methods for the preparation of the compositions according to techniques well known in the art of formulation such as but not limited to homogenization, high speed homogenization, high pressure homogenization and/or sonication could be used for the preparation of the nanoparticles of the in oil encapsulated vitamin E and the seawater, in addition to lyophilisation, grinding, blending, siering, processing, packing.

The invention further provides the methods for the preparation of the water poor/ insoluble parts of fruits, plants and vegetables for further processing of the compositions according to this invention.

The invention further provides the methods for the use and application of the compositions characterized in that the intake and/or administration preferably selected from water solution, lyophilized powder, lyophilized mixture, dried mixture as powder, dried homogenized mixture, emulsion, solid, liquid, suspension, emulsion and/or any other formulation. The composition according to this patent could be prepared with seawater, with olive oil.

The compositions according to this invention could be packaged in airtight sealing or not; sterile solution for oral and/or parenteral use, granulates and any appropriate formulation for use not limited to bottles, cap of bottles, cups, cap of cups, containers, bags, sachets, effervescent tablets, packaging units could be equipped with spoon or straw pipe.

Probiotics, enzymes and coenzyme are sensitive to acid and run the risk of being rendered less effective while being in contact with different substances. One way to allow for these ingredients to be kept fresh until the moment of consumption is to utilize a fully locked, dispenser cap, in which these substances will remain in a dry state, until the consumer decides to take the product. At this point in time, when the consumer will activate the cap by twisting the top, the dry ingredients are dropped into the second part of the container where the liquid mixture of seawater, plant extracts, vegetables, etc. has been stored. The final product is ready to be consumed, with the highest number of nutrients possible.

Also contemplated herein is a kit comprising one or more compositions according to this invention.

The invention involves the surprising and unexpected teaching that the compositions are providing optimal balancing of nutrition including the minerals, trace elements, vitamins, enzymes, and coenzymes.

### DETAILED DESCRIPTION OF THE INVENTION

**It should be understood that the purposes of the present invention, the following terms have the following meanings:**
The terms "seawater" is defined for the purpose of the present invention as seawater as a storehouse of chemicals and contains preferably more than 73 elements in dissolved state. As H. Ohya, T. Suzuki and S. Nakao stated, almost all elements in the periodic table can be found in seawater although many are at very low concentrations*. The main ions which make up 99.9% of the salts in seawater in decreasing order are: Na+ > Mg2+ > Ca2+, K+ > Sr2+ (for cations) and Cl- > SO42- > HCO3- > Br- > BO32- > F- (for anions).

The terms "Fresh water" is defined for the purpose of the present invention as is what we drink and also use for agriculture. In many instances the water is severely deficient in nutritious minerals, particularly the water obtained from desalination plants. Indeed, it is unfortunate that the desalination process not only drives out sodium chloride but also essential minerals and in cases such as reverse osmosis-based desalination, these are preferentially driven out making a bad situation worse.

The terms "oils" is defined for the purpose of the present invention as major oils: coconut oil, corn oil, canola oil, olive oil, palm oil, peanut oil, safflower oil, sesame oil, soybean oil, sunflower oil. Nut oils: almond oil, cashew oil, hazelnut oil, macadamia oil, pecan oil, pistachio oil, walnut oil, food supplements : acai oil, blackcurrant seed oil, borage seed oil, evening primrose oil, Other edible oils: amaranth oil, apricot oil, argan oil, artichoke oil, avocado oil, babassu oil, ben oil, borneo tallow nut oil, buffalo gourd oil, carob pod oil, coriander seed oil, false flax oil, grape seed oil, hemp oil, kapok seed oil, lallemantia oil, meadowfoam seed oil. mustard oil (pressed), okra seed oil (hibiscus seed oil), perilla seed oil, pequi oil, pine nut oil. poppy seed oil, prune kernel oil, pumpkin seed oil, quinoa oil, ramtil oil, rice bran oil, tea oil (camellia oil, thistle oil).

The terms "lyophilization" is defined for the purpose of the present invention as a method of drying food or blood plasma or or pharmaceuticals or tissues without destroying their physical structure; material is frozen and then warmed in a vacuum so that the ice sublimes Properties of freeze-dried products

Freeze-drying also causes less damage to the substance than other dehydration methods using higher temperatures. Freeze-drying does not usually cause shrinkage or toughening of the material being dried. In addition, flavors, smells and nutritional content generally remain unchanged, making the process popular for preserving food.

Freeze-dried products can be rehydrated (reconstituted) much more quickly and easily because the process leaves microscopic pores. The pores are created by the ice crystals that sublimate, leaving gaps or pores in their place. This is especially important when it comes to pharmaceutical uses. Freeze-drying can also be used to increase the shelf life of some pharmaceuticals for many years.

The terms "homogenization" is defined for the purpose of the present invention as a process to make uniform or similar, as in composition, sonication, high speed and high pressure hommogenizers are industrial equipment devices used to achieve homogenization.

The terms "emulsion" is defined for the purpose of the present invention as either a water-in-oil (w/o) or oil-in-water (o/w) emulsion. Regardless of the fact that both types are majorly made up of the same two compounds, each is distinct in its chemical properties, applications, and more.

The most significant, difference between oil-in water (o/w) and water-in oil (w/o) emulsions is which phase is suspended and which is continuous. Oil and water are normally immiscible, but with proper mixing and stability agents, a permanent mixture, or emulsion, can be achieved. O/w emulsions are comprised of oil droplets suspended in an aqueous phase, while w/o emulsions are the opposite- water droplets suspended in a continuous oil phase. Smaller droplet sizes will enhance the effectiveness of either system; this may translate to increased bioavailability in pharmaceutical/food supplement products or extended shelf life in food/beverage products.

The terms "vehicles" is defined for the purpose of the present invention as the oil droplets with the encapsulated water soluble minerals, trace elements , vitamins in the emulsion. The droplets according to the invention could form nano - and macro-particles.

The terms "agitation" is defined for the purpose of the present invention as mechanical or air agitation using appropriate type of mixers to disperse components in multiphase mixtures, to improve heat and mass transfer and to develop new structures.

The terms "selenium" is defined for the purpose of the present invention as selenium, any salt, complex of selenium, and/or conjugates. Selenium is a component of glutathione peroxidase, maintains the function of the cell membrane through oxidation, and increase the production of protein lipid based endogenous antioxidants.

The terms "dietary and nutritional supplements" is defined for the purpose of the present invention as dietary and nutritional supplements; nutritional supplements; nutritional supplements for veterinary use; nutritional supplements for livestock feed; mineral nutritional supplements; delivery agents in the form of coatings for tablets that facilitate the delivery of nutritional supplements; delivery agents in the form of dissolvable films that facilitate the delivery of nutritional supplements; dietary supplements for animals; anti-oxidant food supplements; dietary supplements for humans; dietetic beverages adapted for medical purposes; dietary supplements for pets in the nature of a powdered drink mix; dietary supplements for infants; dietary supplements consisting of vitamins; food supplements consisting of trace elements; Food supplements for non-medical purposes; nutritional supplement meal replacement bars for boosting energy; nutraceuticals for use as a dietary supplement; vitamin and mineral supplements; prenatal minerals and vitamins; minerals and vitamin supplements; mineral and vitamin preparations.

The terms "non-alcoholic drinks" is defined for the purpose of this patent as; Isotonic non-alcoholic drinks; non-alcoholic fruit drinks; carbonated non-alcoholic drinks; non-alcoholic vegetable juice drinks; non-alcoholic carbonated beverages; vitamin fortified non-alcoholic beverages; non-alcoholic honey-based beverages; non-alcoholic fruit extracts used in the preparation of beverages; essences for making non-alcoholic drinks, not in the nature of essential oils.

The term "suspension" is defined for the purpose of this patent as a heterogeneous mixture in which the solute particles do not dissolve, but get suspended throughout the bulk of the solvent, left floating around freely in the medium.^{[1]} The internal phase (solid) is dispersed throughout the external phase (fluid) through mechanical agitation, with the use of certain excipients or suspending agents.

The term "nanoparticles" is defined for the purpose of this patent as an ultrafine unit with dimensions measured in nanometres (nm; 1 nm = 10⁻⁹ metre). "Microparticles" are particles between 0.1 and 100 µm in size.

There are three major physical properties of nanoparticles, and all are interrelated: (1) they are highly mobile in the free state (e.g., in the absence of some other additional influence, a 10-nm-diameter nanosphere of silica has a sedimentation rate under gravity of 0.01 mm/day in water); (2) they have enormous specific surface areas (e.g., a standard teaspoon, or about 6 ml, of 10-nm-diameter silica nanospheres has more surface area than a dozen doubles-sized tennis courts; 20 percent of all the atoms in each nanosphere will be located at the surface); and (3) they may exhibit what are known as quantum effects. In addition, nanoparticles can be classified as hard (e.g., titania[titanium dioxide], silica [silica dioxide] particles, and fullerenes) or as soft (e.g., liposomes, vesicles, and nanodroplets). Thus, nanoparticles have a vast range of compositions, depending on the use or the product.

The term "vitamin E" is defined for the purpose of this patent as alpha-tocopherol, an antioxidant vitamin which binds oxygen free radicals that can cause tissue damage. Deficiency of vitamin E can lead to anemia. Vitamin E may play a possible role in preventing heart disease and cancer of the lung and is a preferred vitamin of the present invention.

Fat-soluble (oil-soluble) vitamins are preferably vitamins A, D, E and/or K. Water-soluble vitamins are preferably B vitamins -- folate, thiamine, riboflavin, niacin, pantothenic acid, biotin, vitamin B6 and vitamin B12 -- and vitamin C.

The term "transmucosal" is defined for the purpose of this patent as the route for entering through, or across, a mucous membrane, as the administration of a drug via the cavity between the cheek and gum.

The term "buccal administration" is defined for the purpose of this patent as a topical route of administration by which drugs held or applied in the buccal area (in the cheek) diffuse through the oral mucosa (tissues which line the mouth) and enter directly into the bloodstream. Buccal administration may provide better bioavailability of some drugs and a more rapid onset of action compared to oral administration because the medication does not pass through the digestive system and thereby avoids first pass metabolism.

In pharmacology, the term "bioavailability (BA or F)" is defined for the purpose of this patent as a subcategory of absorption and is the fraction of an administered dose of unchanged drug that reaches the systemic circulation, one of the principal pharmacokinetic properties of drugs. By definition, when a medication or food supplement is administered intravenously, its bioavailability is 100%. However, when a medication is administered via other routes (such as orally), its bioavailability generally decreases (due to incomplete absorption and first-pass metabolism) or may vary from patient to patient. Bioavailability is one of the essential tools in pharmacokinetics, as bioavailability must be considered when calculating dosages for non-intravenous routes of administration.

For dietary supplements, herbs and other nutrients in which the route of administration is nearly always oral, bioavailability generally designates simply the quantity or fraction of the ingested dose that is absorbed.

Bioavailability is defined slightly differently for drugs as opposed to dietary supplements primarily due to the method of administration and Food and Drug Administration regulations.

The terms "dispersant" or "dispersing agent" or "plasticizer" or "superplasticizer" are defined for the purpose of this patent as either a non-surface active polymer or a surface-active substance added to a suspension, usually a colloid, to improve the separation of particles and to prevent settling or clumping. Dispersants consist normally of one or more surfactants.

The term "agitation" is defined for the purpose of this patent as the action of putting into motion by shaking or stirring, often to achieve mixing.

The term "homogenization" or "homogenization" is defined for the purpose of this patent as any of several processes used to make a mixture of two mutually non-soluble liquids the same throughout. This is achieved by turning one of the liquids into a state consisting of extremely small particles distributed uniformly throughout the other liquid. A typical example is the homogenization of milk, where the milk fat globules are reduced in size and dispersed uniformly through the rest of the milk.

The term "sonication" is defined for the purpose of this patent as the act of applying sound energy to agitate particles in a sample, for various purposes such as the extraction of multiple compounds from plants, microalgae and seaweeds. Ultrasonic frequencies (>20 kHz) are usually used, leading to the process also being known as ultrasonication or ultra-sonication.

In chemistry, the term "salts" is defined for the purpose of this patent as an ionic compound that can be formed by the neutralization reaction of an acid and a base. Salts are composed of related numbers of cations (positively charged ions) and anions (negative ions) so that the product is electrically neutral (without a net charge). These component ions can be inorganic, such as chloride (Cl⁻), or organic, such as acetate (CH₃CO⁻₂); and can be monatomic, such as fluoride (F⁻), or polyatomic, such as sulfate (SO²⁻₄).

Types of salts: Salts can be classified in a variety of ways. Salts that produce hydroxide ions when dissolved in water are called alkali salts. Salts that produce acidic solutions are acidic salts. Neutral salts are those salts that are neither acidic nor basic. Zwitterions contain an anionic and a cationic centres in the same molecule, but are not considered to be salts. Examples of zwitterions include amino acids, many metabolites, peptides, and proteins.

The term "essences" is defined for the purpose of this patent as
a substance obtained from a plant, drug, or the like, by distillation, infusion, etc., and containing it s characteristicproperties in concentrated form.

The term "plant extract" is defined for the purpose of this patent as a substance or an active with desirable properties that is removed from the tissue of a plant, usually by treating it with a solvent, to be used for a particular purpose. Extracts may be used in various sectors of activities: Food and functional properties for foodstuffs (antioxidant, texturizer and others), processing aids, additives - chemical replacers, pharmaceutical for therapeutic properties - preventive and/or curative - cosmetic for functional properties for beauty and well-being.

Extraction is a process with one or more of the following steps: pressing, distillation and hydro-distillation, solid/liquid extraction (maceration, digestion, decoction, infusion, elution, leaching, lixiviation, percolation.

The term "aromatic compound" is defined for the purpose of this patent as any of a large class of unsaturated chemical compounds characterized by one or more planar rings of atoms joined by covalent bonds of two different kinds.

The term "officinal drugs, plants and herbs" is defined for the purpose of this patent as those which are sold in a chemist or druggist shop. Officinal medical preparations of such drugs are made in accordance with the prescriptions authorized by a pharmacopoeia.

The term "biologic" is defined for the purpose of this patent as it is manufactured in a living system such as a microorganism, or plant or animal cells. Most biologics are very large, complex molecules or mixtures of molecules. Many biologics are produced using recombinant DNA technology.

The term "not biological" is defined for the purpose of this patent as not involving or derived from biology or living organisms.

The term "Lyophilization" is defined for the purpose of this patent as the process
of preserving something by freezing it very quickly and then subjecting it to a vacuum which removes ice.

Lyophilization is one of the most effective methods for the long-term preservation of cells. Freeze drying, known as lyophilization, may be used to prepare a dosage form that is to be reconstituted for injection.

The term "granulate" is defined for the purpose of this patent as the process to form or crystallize into grains or granules.

The term "granule" is defined for the purpose of this patent as a small particle; especially: one of numerous particles forming a larger unit
The term "appropriate formulations for packaging" is defined for the purpose of this patent such as the formulations that are orally, parenteral, selected for the composition to be packaged for the protection from moisture, light, and the prevention of the extractable substances.

The term "food preservatives" is defined for the purpose of this patent as substances that under certain conditions, either delay the growth of microorganisms without necessarily destroying them or prevent deterioration of quality during manufacture and distribution.

The term "isotonic sodium choloride solution" is defined for the purpose of this patent as
a solution of a salt or salts that is essentially isotonic with tissue fluids or blood; especially: an approximately 0.9 percent solution of sodium chloride - called also normal saline solution, normal salt solution, physiological saline solution, physiological salt solution.

The term "enzyme" is defined for the purpose of this patent as a substance produced by a living organism which acts as a catalyst to bring about a specific biochemical reaction.

The term "co-enzyme" is defined for the purpose of this patent as a substance that enhances the action of an enzyme. (An enzyme is a protein that functions as a catalyst to mediate and speed a chemical reaction).

Coenzymes are small molecules. They cannot by themselves catalyze a reaction but they can help enzymes to do so. In technical terms, coenzymes are organic nonprotein molecules that bind with the protein molecule (apoenzyme) to form the active enzyme (holoenzyme).

A number of the water-soluble vitamins such as vitamins B1, B2 and B6 serve as coenzymes.

The term "probiotics" is defined for the purpose of this patent a substance or preparation. They are microorganism introduced into the body for its beneficial qualities. They are live bacteria and yeasts that are good for the body.

The term "prebiotics" is defined for the purpose of this patent as food ingredients that induce the growth or activity of beneficial microorganisms (e.g., bacteria and fungi). The most common example is in the gastrointestinal tract, where prebiotics can alter the composition of organisms in the gut microbiome.

Compounds that can be classified as prebiotics must also meet the following criteria:
- non-digestible and resistant to breakdown by stomach acid and enzymes in the human gastrointestinal tract
- selectively fermented by intestinal microorganisms
- selectively target and stimulate the growth and activity of beneficial bacteria

The term "trace elements" is defined for the purpose of this patent as any chemical element required by living organisms in minute amounts (that is less than 0.1 percent by volume [1,000 parts per million]), usually as part of a vital enzyme (a cell-produced catalytic protein).

Exact needs vary among species, but commonly required plant trace elements include copper, boron, zinc, manganese, and molybdenum. Animals also require manganese, iodine, and cobalt. Lack of a necessary plant trace element in the soil causes deficiency diseases; lack of animal trace elements in the soil may not harm plants, but, without them, animals feeding solely on those plants develop their own deficiency diseases.

Essential trace elements of the human body include zinc (Zn), copper (Cu), selenium (Se), chromium (Cr), cobalt (Co), iodine (I), manganese (Mn), and molybdenum (Mo). Although these elements account for only 0.02% of the total body weight, they play significant roles, e.g., as active centers of enzymes or as trace bioactive substances. A major outcome of trace element deficiencies is reduced activity of the concerned enzymes. However, since each trace element is related to so many enzymes, deficiency of a single trace element is often not associated with any specific clinical manifestations, but rather manifests as a combination of various symptoms. Because of the presence of trace elements in very small amounts and the absence of specific clinical features associated with their deficiency, it is often difficult for clinicians to identify deficiencies of some particular trace elements.

The term "oral use" is defined for the purpose of this patent as the route of administration where a substance is taken through the mouth. Many medications and food supplements are taken orally because they are intended to have a systemic effect, reaching different parts of the body via the bloodstream, for example.

Oral administration is a part of enteral administration, which also includes
- Buccal, dissolved inside the cheek
- Sublabial, dissolved under the lip
- Sublingual administration, dissolved under the tongue, but due to rapid absorption many consider SL a parenteral route

The term "administration of oral formulations" is defined for the purpose of this patent as:
1.Granulate compound to be dissolved sublingual
2.Soluble tablets
3.Saquetes (like tea) to be dissolved in water or other drinkable liquid
4.Flavoring for soups
   - Mix with spices for food flavoring
   - Vegetable concentrates used for seasoning
   - To use it as another ingredient in meal preparation
   - To be added to already commercialized sports drinks
   - To be added to already finished meal
   - To be added to cereals, either as loose cereals, or in a cereal bar format
   - Mouth wash and mouth spray

The term "administration of non-oral formulations" is defined for the purpose of this patent as:
- Usage of the product as "bath salts" to have a revitalizing bath
- To use it as an ingredient with a shampoo type of product for hair treatment to prevent hair loss, or stand alone solution
- Body spray to nurture skin

The term "non oral use" is defined for the purpose of this patent as the route of administration of any substance that is not done through the mouth.

The term "parenteral" is defined for the purpose of this patent as the route of administration that can be performed by injection, that is, using a needle (usually a hypodermic needle) and a syringe, or by the insertion of an indwelling catheter.

The term "topical" is defined for the purpose of this patent as an agent which is applied to a certain area of the skin and is intended to affect only the area to which it is applied. Whether its effects are indeed limited to that area depends on whether the agent stays where it is put or is absorbed into the bloodstream.

The term "inhalation" is defined for the purpose of this patent as the process or act of breathing in, taking air and sometimes other substances into your lungs.

The term "spray drying" is defined for the purpose of this patent as a method of producing a dry powder from a liquid or slurry by rapidly drying with a hot gas. This is the preferred method of drying of many thermally-sensitive materials such as foods and pharmaceuticals.

The term "spray solution" is defined for the purpose of this patent as liquid that is blown or driven through the air in the form of tiny drops.

The term "nutraceutical" is defined for the purpose of this patent such as foods having a medicinal effect on health of human beings. It consists of food supplements, herbal products, probiotics and prebiotics, medical foods meant for prevention and treatment of diseases. Major nutraceuticals posses multiple therapeutic effect with lacking of unwanted effects hence attract more consumer interest.

The term "nasal" is defined for the purpose of this patent as the route of administration pertaining to the nose.

The term "transdermal" is defined for the purpose of this patent as the route of administration wherein active ingredients are delivered across the skin for systemic distribution.

### FIGURES

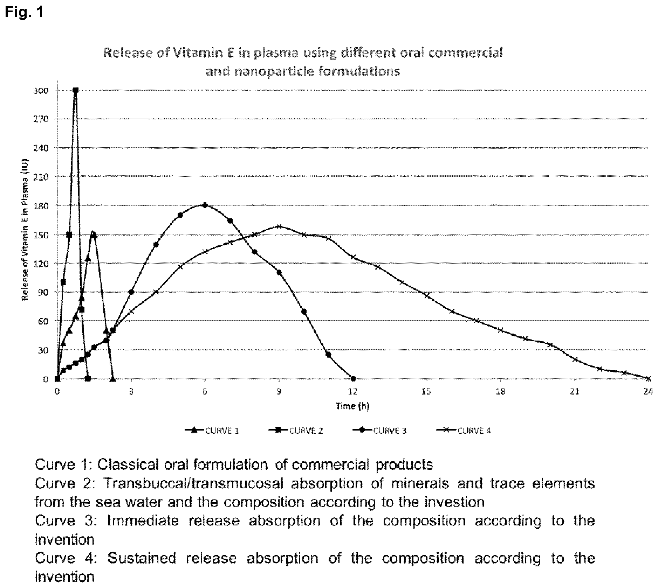

FIG. 1: Release of Vitamin E in plasma using different oral commercial and nanoparticle formulations.

The invention is further described by way of illustrative examples

### EXAMPLES

### Examples for the preparation of the composition comprising (i) seawater together with vitamin E, olive oil, ozonated olive oil, coconut oil, hemp oil, and/or jojoba oil

### Examples of Vitamin E and /or oil nanoparticles Preparation

### Example 1- General Procedure

In order to prepare the nanoparticle composition from vitamin E and/or other oils an injection method may, for instance, be applied where the aqueous phase, which can contain the water (natural, artificial seawater, mineral water) to be encapsulated, is agitated to speed up the encapsulation process of the trace amounts of minerals, elements, ions and metals forming nanoparticles. Depending on solubility, the substance to be encapsulated can be located either in the aqueous or in the lipid phase. It depends on the conditions of preparation, such as the agitation rate, injection rate and temperature, whether uni-lamellar and/or multilamellar and/or heterolamellar, small and/or large nano - particles are obtained.

### Example 2- General Procedure

The following procedures are further example for the preparation of the composition according to this patent:
1. Mix Vitamin E and/or the oil(s).
2. Add this mixture gradually in small quantities, over a period of one hour to the seawater.
3. The mixture will be brought to a temperature of 50C° under agitation.
4. Record all conditions e.g. temperature, time for any process such as vortex, agitation, homogenization etc.
5. High speed homogenization, high pressure homogenization and/or sonication could be used for the preparation of the nanoparticles of the in oil encapsulated vitamin E and the seawater.

### Example 3

According to examples 1 and 2 of this patent a mixture of liquid olive oil and/or solid paste of ozonated olive oil was used.

### Vitamin E, olive oil, coconut oil, hemp oil, and/or jojoba oil

1. Mix Vitamin E and the oil(s).
2. Add this mixture gradually in small quantities, over a period of one hour to the seawater prepared from seawater powder and seawater or mineral water.
3. The mixture will be brought to a temperature of 50C° under agitation.
4. The mixture will be vortexed, agitated and/or homogenized (high speed homogenization or high pressure homogenization) and/or sonicated. Record all conditions e.g. temperature, time for any process such as vortex, agitation, homogenization, sonication etc.
5. High speed homogenization, high pressure homogenization and/or sonication could be used for the preparation of the nanoparticles of the in oil encapsulated vitamin E and the seawater.
6. Add this emulsion to the paste of ozonated olive gradually.
7. The mixture will be homogenized.
8. The formulation will be used for oral application.

### Results

It is surprisingly and unexpected that the release profile of Vitamin E in olive oil composition is different than the composition without the olive oil and/or oils according to this example.

The fast-release of Vitamin E in the oil formulation is surprisingly and unexpected faster due to the transmucosal fast absorption of the encapsulated Vitamin E in the lipid oil vesicles.

In addition, figure 1 shows the different release profiles of Vitamin E in plasma, using the following formulations: (1) Classical oral formulation of commercial products, (2) transbuccal/transmucosal absorption of minerals and trace elements from the seawater and the composition according to the investion, (3) Immediate release absorption of the composition according to the invention, (4) Sustained release absorption of the composition according to the invention.

**The examples 4-9 according to this patent will be used as follows but not limited to:**
**Seawater:** as seawater or seawater salt or as powder or mineral water.
**Ingredients** for the preparation of the composition according to examples 6-11 of this patent. They are applied as single or in mixture, prepared and displayed by individual typologies or not.
   1. Salts, minerals, trace elements, plants, plant extracts, vitamins, enzymes and co-enzymes
   2. Fruits in all their parts
   3. Probiotics
   4. Olive oil could be used in any of the examples 6-11 as ingredient
   5. Additives such as but not limited to as essences, aromatics, officials, biologic and not biologic
**The composition** according the examples 6-11 as:
   1. Seawater solution
   2. Lyophilysat powder
   3. Lyophilized mixture
   4. Dried mixture as powder
   5. Dried mixture
   6. Homogenized mixture
   7. Emulsion
   8. Solid
   9. Liquid
   10. Suspension
   11. Emulsion and/or any other
   12. Composition according to this patent could be prepared with or without seawater, with or without olive oil.
   The compositions of the present patent according to the examples 6-11 are prepared according to techniques well known in the art of formulations selected for these applications. They may be prepared employing suitable preservatives, absorption promoters to enhance bioavailabiliy, solubilizing or dispersing agents known in the art.
**Packaging:** In airtight sealing or not; sterile solution for oral and/or parenteral use, granulates and any appropriate formulation for use.

Packaging units could be equipped with spoon or straw pipe.
1. Bottles
2. Cups
3. Containers
4. Bags
5. Sachets
6. Effervescent tablets

### Example 4

### Preparation of compositions comprising of:

1. Seawater
2. Manganese
3. Calcium
4. Selenium
5. Potassium
6. Maca
7. Rhodiola
8. Schisandra
9. Probiotic
10. Co-enzyme Q10
11. Vitamin C
12. Vitamin D

### Example 5

**Preparation of compositions comprising of** :
1. Seawater
2. Cardomom
3. Anis
4. Flaxseeds
5. Probiotic
6. Co-enzyme Q10
7. Iron
8. Zinc
9. Selenium
10. Manganese
11. Vitamin A
12. Vitamin B6
13. Vitamin B12
14. Vitamin C
15. Vitamin E

### Example 6

**Preparation of compositions comprising of:**
1. Seawater
2. Guarana
3. Red Yeast rice
4. Green Tea
5. Probiotic
6. Co-enzyme Q10
7. Zinc
8. Selenium
9. Calcium
10. Manganese
11. Vitamin C
12. Vitamin B6
13. Vitamin B12

### Example 7

### Preparation of fruit compositions

Fruit compositions according to the patent and this example will be prepared from each of the fruits or of the mixture as: lyophilysat powder, lyophilized mixture, homogenized mixture, dried mixture, dried mixture as powder, oral solution, effervescent granulates, effervescent tablets, tablets.
1. Seawater
2. Raspberries
3. Strawberries
4. Blackberries
5. Blueberries
6. Sour Cherries
7. Apricots
8. Apples
9. Aronia Berries
10. Redcurrants
11. Plums
12. Lemons
13. Pears
14. Kiwis
15. Peaches
16. Banana
17. Pineapple

### Example 8

1. Seawater
2. Manganese
3. Calcium
4. Selenium
5. Potassium
6. Maca
7. Rhodiola
8. Schisandra
9. Probiotic
10. Co-enzyme Q10
11. Vitamin C
12. Vitamin D
   +
18. Raspberries
19. Strawberries
20. Blackberries
21. Blueberries
22. Sour Cherries
23. Apricots
24. Apples
25. Aronia Berries
26. Redcurrants
27. Plums
28. Lemons
29. Pears
30. Kiwis
31. Peaches
32. Banana
33. Pineapple

### Example 9

1. Seawater
2. Cardomom
3. Anis
4. Flaxseeds
5. Probiotic
6. Co-enzyme Q10
7. Iron
8. Zinc
9. Selenium
10. Manganese
11. Vitamin A
12. Vitamin B6
13. Vitamin B12
14. Vitamin C
15. Vitamin E
   +
16. Raspberries
17. Strawberries
18. Blackberries
19. Blueberries
20. Sour Cherries
21. Apricots
22. Apples
23. Aronia Berries
24. Redcurrants
25. Plums
26. Lemons
27. Pears
28. Kiwis
29. Peaches
30. Banana
31. Pineapple

### Example 10

1. Seawater
2. Guarana
3. Red Yeast rice
4. Green Tea
5. Probiotic
6. Co-enzyme Q10
7. Zinc
8. Selenium
9. Calcium
10. Manganese
11. Vitamin C
12. Vitamin B6
13. Vitamin B12
   +
14. Raspberries
15. Strawberries
16. Blackberries
17. Blueberries
18. Sour Cherries
19. Apricots
20. Apples
21. Aronia Berries
22. Redcurrants
23. Plums
24. Lemons
25. Pears
26. Kiwis
27. Peaches
28. Banana
29. Pineapple
**30.**

### Example 11

**The examples 13 according to this patent will be used as follows but not limited to:**
**Seawater:** as seawater or seawater salt or as powder or mineral water.
**Ingredients** for the preparation of the composition according to these examples of this patent are applied as single or in mixture, of vegetables prepared and displayed by individual typologies or not.
   1. Salts, minerals, trace elements, vitamins, plants, plant extracts, vitamins, enzymes and co-enzymes
   2. Organic and non organic Vegetables in all their parts: external vegetative part, roots, flowers and biologic and non biologic organic fruits
   3. Olive oil could be used in any of the examples 4-9 as ingredient
   4. Additives such as but not limited to as essences, aromatics, officials, biologic and non biologic
**The composition** according the patent example as any of these formulations:
   1. Water solution,
   2. Lyophilysat powder
   3. Lyophilized mixture
   4. Dried mixture as powder
   5. Dried mixture
   6. Homogenized mixture
   7. Emulsion
   8. Solid
   9. Liquid
   10. Suspension
   11. Emulsion and/or any other

Composition according to this patent could be prepared with or without seawater, with or without olive oil.

The compositions of the present patent for the preparation of the vegetables dried powder will be prepared according to techniques well known in the art of formulations and may be prepared as powder and or homogenized mixture employing suitable preservatives, solubility promoters and procedures, absorption promoters to enhance bioavailability, solubilizing or dispersing agents known in the art.

**Packaging:** In airtight sealing or not; sterile solution for oral and/or parenteral use, granulates and any appropriate formulation for use.

Packaging units could be equipped with spoon or straw pipe.
1. Bottles
2. Cap of bottles
3. Cups
4. Cap of cups
5. Containers
6. Bags
7. Sachets
8. Effervescent tablets

### Example 12 - Preparation of Selenium and selenite salts compositions and products

Compositions of the present patent comprising of selenium, selenium salts such as sodium selenite pentahydrate in seawater using seawater, seawater salts as powder, water for injection, or distilled water with or without other ingredients employing suitable preservatives, absorption promoters to enhance bioavailability, solubilizing or dispersing agents known in the art are prepared according to techniques well known in the art of preparation and formulations for oral and/or sterile parenteral and/or topical applications and/or sublingual formulations/ administration.

Selenium and selenite salts of the present patent and example 14 will be manufactured according to the processes and procedures known in the art. The compositions could be manufactured, but not limited to, as tablets, capsules, dragées, sublingual tablets, granules, or oral solution as single and/or multiple dose. The content of the selenium/dosage strength could be for example in the range of 10-50 microgram, 100mcg, 200mcg, 300 mcg or 400 mcg or higher per tablet, capsules, dragées, sublingual tablet, or 1ml or 2ml ampoules for oral intake.

## Claims

1. Composition as a dietary and nutritional supplement in an oral administration form, comprising:
(1) seawater,
(2) one or more minerals, trace elements, vitamins, prebiotics, probiotics, enzymes, co-enzymes, parts of a plant and/or plant extracts, comprising at least one oil-soluble vitamin, and
(3) one or more oils,
wherein said composition is obtained using a nanoparticle method, said method comprising homogenizing and/or sonicating a seawater and oil mixture, such that the composition comprises liposomes or vesicles of nanoparticle size, or mixtures thereof.

2. Composition according to the preceding claim, wherein the particle method for the preparation of the composition comprises:
a. Mixing one or more items of component (2) according to claim 1, comprising an oil-soluble vitamin, with one or more oil(s);
b. Adding the oil mixture from step a. gradually to seawater, optionally mixed with additional items of component (2) according to claim 1, thereby mixing the oil mixture from step a. with seawater,
wherein the gradual adding occurs by adding 10-35% of the oil mixture from step a. every 10-15 minutes over a period of 30 to 150 minutes, preferably approximately 45 to 75 minutes, more preferably approximately 60 minutes;
c. Bringing the temperature of the mixture in step b. to 40 to 60 degrees Celsius, preferably approximately 50 degrees Celsius; either during and/or after the mixing in step b.,
d. Homogenizing and/or sonicating the mixture in step b., either during and/or after the mixing in step b.; and
e. Formulating the mixture after step d. to a composition in the form of a lyophilized powder, tablet, capsule, dragée, sublingual tablet, granule, emulsion or suspension suitable for oral administration.

3. Composition according to any one of the preceding claims, wherein the composition is prepared as a dried mixture.

4. Composition according to any one of the preceding claims, wherein the composition comprises:
a. one or more minerals and/or trace elements; and
b. one or more vitamins, enzymes and/or co-enzymes.

5. Composition according to any one of the preceding claims, wherein the composition comprises:
a. one or more minerals and/or trace elements;
b. one or more vitamins, enzymes and/or co-enzymes; and
c. one or more parts of a plant or plant extract, preferably parts of a fruit or vegetable or fruit or vegetable extract.

6. Composition according to any one of the preceding claims, wherein the composition comprises:
a. one or more minerals and/or trace elements;
b. one or more vitamins, enzymes and/or co-enzymes;
c. one or more parts of a plant or plant extract, preferably parts of a fruit or vegetable or fruit or vegetable extract; and
d. one or more prebiotics and/or probiotics.

7. Composition according to any one of the preceding claims, wherein the one or more minerals and/or trace elements are selected from iron, zinc, manganese, calcium, selenium and/or potassium.

8. Composition according to any one of the preceding claims, wherein the one or more parts of a plant or plant extract are selected from maca, rhodiola and/or Schisandra, and/or the one or more parts of a plant or plant extract are selected from guarana, red yeast rice, green tea.

9. Composition according to any one of the preceding claims, wherein the one or more vitamins, enzymes and/or co-enzymes are selected from co-enzyme Q10, vitamin C, D, A, B6, B12, D3, C, and/or vitamin E.

10. Composition according to any one of the preceding claims, wherein the one or more parts of a plant or plant extract, preferably parts of a fruit or vegetable or fruit or vegetable extract, are selected from raspberries, strawberries, blackberries, blueberries, sour cherries, apricots, apples, aronia berries, red currants, plums, lemons, pears, kiwis, peaches, banana, and/or pineapple.

11. Composition according to any one of the preceding claims, wherein the composition comprises particles in the form of vesicles comprising vitamin E, and olive oil, in addition to at least one mineral, trace element and/or vitamin.

12. Composition according to any one of the preceding claims, wherein the composition comprises:
a. at least one of manganese, calcium, selenium, potassium,
b. at least one of maca, rhodiola, schisandra, probiotic,
c. at least one of co-enzyme Q10, vitamin C, D, and
d. at least one of raspberries, strawberries, blackberries, blue berries, sour cherries, apricots, apples, aronia berries, red currants, plums, lemons, pears, kiwis, peaches, banana, and/or pineapple.
or wherein the composition comprises:
a. at least one of cardamom, anise, flaxseeds, probiotics,
b. at least one of Co-enzyme Q10, vitamin A, B6, B12, D3, C, E,
c. at least one of iron, zinc, selenium, manganese, and
d. at least one of raspberries, strawberries, blackberries, blueberries, sour cherries, apricots, apples, aronia berries, red currants, plums, lemons, pears, kiwis, peaches, banana, and/or pineapple.
or wherein the composition comprises:
a. at least one of guarana, red yeast rice, green tea, probiotic,
b. at least one of co-enzyme Q10, vitamin C, B6, B12, D3,
c. at least one of zinc, selenium, calcium, manganese, and
d. at least one of raspberries, strawberries, blackberries, blueberries, sour cherries, apricots, apples, aronia berries, red currants, plums, lemons, pears, kiwis, peaches, banana, and/or pineapple.

13. Method for manufacturing a composition as a dietary and nutritional supplement in an oral administration form, comprising:
(1) seawater,
(2) one or more minerals, trace elements, vitamins, prebiotics, probiotics, enzymes, co-enzymes, parts of a plant and/or plant extracts, comprising at least one oil-soluble vitamin, and
(3) one or more oils,
wherein said method comprises a nanoparticle method comprising homogenizing and/or sonicating a seawater and oil mixture, such that the composition comprises liposomes or vesicles of nanoparticle size, or mixtures thereof.

14. Method according to the preceding claim, comprising:
a. Mixing one or more items of component (2) according to claim 14, comprising an oil-soluble vitamin, with one or more oil(s);
b. Adding the oil mixture from step a. gradually to seawater, optionally mixed with additional items of component (2) according to claim 14, thereby mixing the oil mixture from step a. with seawater,
wherein the gradual adding occurs by adding 10-35% of the oil mixture from step a. every 10-15 minutes over a period of 30 to 150 minutes, preferably approximately 45 to 75 minutes, more preferably approximately 60 minutes;
c. Bringing the temperature of the mixture in step b. to 40 to 60 degrees Celsius, preferably approximately 50 degrees Celsius; either during and/or after the mixing in step b.,
d. Homogenizing and/or sonicating the mixture in step b., either during and/or after the mixing in step b.; and
e. Formulating the mixture after step d. to a composition in the form of a lyophilized powder, tablet, capsule, dragée, sublingual tablet, granule, emulsion, suspension, or composition suitable for oral administration.

15. Method according to any on of claims 13 or 14, wherein the composition is prepared as a dried mixture.

## Patentansprüche

1. Zusammensetzung als Ernährungs- und Nahrungsergänzungsmittel in oraler Darreichungsform, umfassend:
(1) Meerwasser,
(2) ein(en) oder mehrere Mineralien, Spurenelemente, Vitamine, Präbiotika, Probiotika, Enzyme, Coenzyme, Teile einer Pflanze und/oder von Pflanzenextrakten, umfassend mindestens ein öllösliches Vitamin, und
(3) ein oder mehrere Öle,
wobei die Zusammensetzung unter Verwendung eines Nanopartikelverfahrens erhalten wird, wobei das Verfahren Homogenisieren und/oder Beschallen eines Meerwasser-ÖlGemisches umfasst, sodass die Zusammensetzung Liposomen oder Vesikel von Nanopartikelgröße oder Gemische davon umfasst.

2. Zusammensetzung nach einem der vorhergehenden Ansprüche, wobei das Partikelverfahren zur Herstellung der Zusammensetzung Folgendes umfasst:
a. Mischen eines oder mehrerer Bestandteile der Komponente (2) nach Anspruch 1, umfassend ein öllösliches Vitamin, mit einem oder mehreren Öl(en);
b. Zugeben des Ölgemisches aus Schritt a. allmählich zu Meerwasser, optional gemischt mit weiteren Bestandteilen der Komponente (2) nach Anspruch 1, wodurch das Ölgemisch aus Schritt a. mit Meerwasser vermischt wird,
wobei das allmähliche Zugeben durch Zugeben von 10-35 % des Ölgemisches aus Schritt a. alle 10-15 Minuten über einen Zeitraum von 30 bis 150 Minuten, vorzugsweise etwa 45 bis 75 Minuten, mehr bevorzugt etwa 60 Minuten erfolgt;
c. Temperieren des Gemisches in Schritt b. auf 40 bis 60 Grad Celsius, vorzugsweise etwa 50 Grad Celsius; entweder während und/oder nach dem Mischen in Schritt b.,
d. Homogenisieren und/oder Beschallen des Gemisches in Schritt b., entweder während und/oder nach dem Mischen in Schritt b.; und
e. Formulieren der Mischung nach Schritt d. zu einer Zusammensetzung in Form eines lyophilisierten Pulvers, einer Tablette, einer Kapsel, eines Dragees, einer Sublingualtablette, eines Granulats, einer Emulsion oder einer Suspension, das bzw. die zur oralen Verabreichung geeignet ist.

3. Zusammensetzung nach einem der vorhergehenden Ansprüche, wobei die Zusammensetzung als ein getrocknetes Gemisch hergestellt wird.

4. Zusammensetzung nach einem der vorhergehenden Ansprüche, wobei die Zusammensetzung Folgendes umfasst:
a. ein oder mehrere Mineralien und/oder Spurenelemente; und
b. ein oder mehrere Vitamine, Enzyme und/oder Coenzyme.

5. Zusammensetzung nach einem der vorhergehenden Ansprüche, wobei die Zusammensetzung Folgendes umfasst:
a. ein oder mehrere Mineralien und/oder Spurenelemente;
b. ein oder mehrere Vitamine, Enzyme und/oder Coenzyme; und
c. einen oder mehrere Teile einer Pflanze oder eines Pflanzenextraktes, vorzugsweise Teile eines Obstes oder Gemüses oder eines Obst- oder Gemüseextrakts.

6. Zusammensetzung nach einem der vorhergehenden Ansprüche, wobei die Zusammensetzung Folgendes umfasst:
a. ein oder mehrere Mineralien und/oder Spurenelemente;
b. ein oder mehrere Vitamine, Enzyme und/oder Coenzyme;
c. einen oder mehrere Teile einer Pflanze oder eines Pflanzenextrakts, vorzugsweise Teile eines Obstes oder Gemüses oder eines Obst- oder Gemüseextrakts; und
d. ein oder mehrere Präbiotika und/oder Probiotika.

7. Zusammensetzung nach einem der vorhergehenden Ansprüche, wobei das eine oder die mehreren Mineralien und/oder Spurenelemente aus Eisen, Zink, Mangan, Calcium, Selen und/oder Kalium ausgewählt sind.

8. Zusammensetzung nach einem der vorhergehenden Ansprüche, wobei der eine oder die mehreren Teile einer Pflanze oder eines Pflanzenextrakts aus Maca, Rhodiola und/oder Schisandra ausgewählt sind, und/oder der eine oder die mehreren Teile einer Pflanze oder eines Pflanzenextrakts aus Guarana, rotem Hefereis, grünem Tee ausgewählt sind.

9. Zusammensetzung nach einem der vorhergehenden Ansprüche, wobei das eine oder die mehreren Vitamine, Enzyme und/oder Coenzyme aus Coenzym Q10, Vitamin C, D, A, B6, B12, D3, C und/oder Vitamin E ausgewählt sind.

10. Zusammensetzung nach einem der vorhergehenden Ansprüche, wobei der eine oder die mehreren Teile einer Pflanze oder eines Pflanzenextrakts, vorzugsweise Teile eines Obstes oder Gemüses oder eines Obst- oder Gemüseextrakts aus Himbeeren, Erdbeeren, Brombeeren, Heidelbeeren, Sauerkirschen, Aprikosen, Äpfeln, Aroniabeeren, roten Johannisbeeren, Pflaumen, Zitronen, Birnen, Kiwis, Pfirsichen, Bananen und/oder Ananas ausgewählt sind.

11. Zusammensetzung nach einem der vorhergehenden Ansprüche, wobei die Zusammensetzung neben mindestens einem Mineral, einem Spurenelement und/oder einem Vitamin Partikel in Form von Vesikeln, die Vitamin E und Olivenöl umfassen, umfasst.

12. Zusammensetzung nach einem der vorhergehenden Ansprüche, wobei die Zusammensetzung Folgendes umfasst:
a. mindestens eines von Mangan, Calcium, Selen, Kalium,
b. mindestens eines von Maca, Rhodiola, Schisandra, Probiotikum,
c. mindestens eines von Co-Enzym Q10, Vitamin C, D und
d. mindestens eines von Himbeeren, Erdbeeren, Brombeeren, Blaubeeren, Sauerkirschen, Aprikosen, Äpfeln, Aroniabeeren, roten Johannisbeeren, Pflaumen, Zitronen, Birnen, Kiwis, Pfirsichen, Bananen und/oder Ananas,
oder wobei die Zusammensetzung Folgendes umfasst:
a. mindestens eines aus Kardamom, Anis, Leinsamen, Probiotika,
b. mindestens eines von Co-Enzym Q10, Vitamin A, B6, B12, D3, C, E,
c. mindestens eines von Eisen, Zink, Selen, Mangan und
d. mindestens eines von Himbeeren, Erdbeeren, Brombeeren, Blaubeeren, Sauerkirschen, Aprikosen, Äpfeln, Aroniabeeren, roten Johannisbeeren, Pflaumen, Zitronen, Birnen, Kiwis, Pfirsichen, Bananen und/oder Ananas,
oder wobei die Zusammensetzung Folgendes umfasst:
a. mindestens eines von Guarana, rotem Hefereis, grünem Tee, Probiotikum,
b. mindestens eines von Co-Enzym Q10, Vitamin C, B6, B12, D3,
c. mindestens eines von Zink, Selen, Calcium, Mangan und
d. mindestens eines von Himbeeren, Erdbeeren, Brombeeren, Blaubeeren, Sauerkirschen, Aprikosen, Äpfeln, Aroniabeeren, roten Johannisbeeren, Pflaumen, Zitronen, Birnen, Kiwis, Pfirsichen, Bananen und/oder Ananas.

13. Verfahren zum Herstellen einer Zusammensetzung als Ernährungs- und Nahrungsergänzungsmittel in oraler Darreichungsform, umfassend:
(1) Meerwasser,
(2) ein(en) oder mehrere Mineralien, Spurenelemente, Vitamine, Präbiotika, Probiotika, Enzyme, Coenzyme, Teile einer Pflanze und/oder von Pflanzenextrakten, umfassend mindestens ein öllösliches Vitamin, und
(3) ein oder mehrere Öle,
wobei das Verfahren ein Nanopartikelverfahren umfasst, das Homogenisieren und/oder Beschallen eines Meerwasser-ÖlGemisches umfasst, sodass die Zusammensetzung Liposomen oder Vesikel von Nanopartikelgröße oder Gemische davon umfasst.

14. Verfahren nach dem vorhergehenden Anspruch, umfassend:
a. Mischen eines oder mehrerer Bestandteile der Komponente (2) nach Anspruch 14, umfassend ein öllösliches Vitamin, mit einem oder mehreren Öl(en);
b. Zugeben des Ölgemisches aus Schritt a. allmählich zu Meerwasser, optional gemischt mit weiteren Bestandteilen der Komponente (2) nach Anspruch 14, wodurch das Ölgemisch aus Schritt a. mit Meerwasser vermischt wird,
wobei das allmähliche Zugeben durch Zugeben von 10-35 % des Ölgemisches aus Schritt a. alle 10-15 Minuten über einen Zeitraum von 30 bis 150 Minuten, vorzugsweise etwa 45 bis 75 Minuten, mehr bevorzugt etwa 60 Minuten erfolgt;
c. Temperieren des Gemisches in Schritt b. auf 40 bis 60 Grad Celsius, vorzugsweise etwa 50 Grad Celsius; entweder während und/oder nach dem Mischen in Schritt b.,
d. Homogenisieren und/oder Beschallen des Gemisches in Schritt b., entweder während und/oder nach dem Mischen in Schritt b.; und
e. Formulieren des Gemisches nach Schritt d. zu einer Zusammensetzung in Form eines lyophilisierten Pulvers, einer Tablette, einer Kapsel, eines Dragees, einer Sublingualtablette, eines Granulats, einer Emulsion, einer Suspension oder einer Zusammensetzung, das bzw. die zur oralen Verabreichung geeignet ist.

15. Verfahren nach einem der Ansprüche 13 oder 14, wobei die Zusammensetzung als ein getrocknetes Gemisch hergestellt wird.

## Revendications

1. Composition comme complément alimentaire et nutritionnel sous forme d'administration orale, comprenant :
(1) l'eau de mer,
(2) un ou plusieurs minéraux, oligo-éléments, vitamines, prébiotiques, probiotiques, enzymes, co-enzymes, parties d'une plante et/ou extraits de plantes, comprenant au moins une vitamine oléosoluble, et
(3) une ou plusieurs huiles,
dans laquelle ladite composition est obtenue à l'aide d'un procédé à base de nanoparticules, ledit procédé comprenant l'homogénéisation et/ou la sonication d'un mélange d'eau de mer et d'huile, de sorte que la composition comprend des liposomes ou des vésicules de taille de nanoparticules, ou des mélanges de ceux-ci.

2. Composition selon la revendication précédente, dans laquelle le procédé particulaire pour la préparation de la composition comprend :
a. le mélange d'un ou de plusieurs éléments de composant (2) selon la revendication 1, comprenant une vitamine oléosoluble, avec une ou plusieurs huile(s) ;
b. l'ajout du mélange d'huile de l'étape a. progressivement à l'eau de mer, éventuellement mélangée à des éléments supplémentaires de composant (2) selon la revendication 1, mélangeant ainsi le mélange d'huiles de l'étape a. avec de l'eau de mer,
dans laquelle l'ajout progressif se produit en ajoutant 10 à 35 % du mélange d'huile provenant de l'étape a. toutes les 10 à 15 minutes sur une période de 30 à 150 minutes, de préférence environ 45 à 75 minutes, de manière davantage préférée environ 60 minutes ;
c. le fait d'amener la température du mélange à l'étape b. à 40 à 60 degrés Celsius, de préférence environ 50 degrés Celsius ; pendant et/ou après le mélange à l'étape b.,
d. l'homogénéisation et/ou la sonication du mélange à l'étape b., pendant et/ou après le mélange à l'étape b. ; et
e. la formulation du mélange après l'étape d. à une composition sous forme de poudre lyophilisée, de comprimé, de gélule, de dragée, de comprimé sublingual, de granule, d'émulsion ou de suspension adaptée à une administration orale.

3. Composition selon l'une quelconque des revendications précédentes, dans laquelle la composition est préparée sous forme d'un mélange séché.

4. Composition selon l'une quelconque des revendications précédentes, dans laquelle la composition comprend :
a. un ou plusieurs minéraux et/ou oligo-éléments ; et
b. une ou plusieurs vitamines, enzymes et/ou co-enzymes.

5. Composition selon l'une quelconque des revendications précédentes, dans laquelle la composition comprend :
a. un ou plusieurs minéraux et/ou oligo-éléments ;
b. une ou plusieurs vitamines, enzymes et/ou coenzymes ; et
c. une ou plusieurs parties d'une plante ou d'un extrait de plante, de préférence des parties d'un fruit ou d'un légume ou d'un extrait de fruit ou de légume.

6. Composition selon l'une quelconque des revendications précédentes, dans laquelle la composition comprend :
a. un ou plusieurs minéraux et/ou oligo-éléments ;
b. une ou plusieurs vitamines, enzymes et/ou coenzymes ;
c. une ou plusieurs parties d'une plante ou d'un extrait de plante, de préférence des parties d'un fruit ou d'un légume ou d'un extrait de fruit ou de légume ; et
d. un ou plusieurs prébiotiques et/ou probiotiques.

7. Composition selon l'une quelconque des revendications précédentes, dans laquelle les un ou plusieurs minéraux et/ou oligo-éléments sont choisis parmi le fer, le zinc, le manganèse, le calcium, le sélénium et/ou le potassium.

8. Composition selon l'une quelconque des revendications précédentes, dans laquelle les une ou plusieurs parties d'une plante ou d'un extrait de plante sont choisies parmi la maca, la rhodiola et/ou la Schisandra, et/ou les une ou plusieurs parties d'une plante ou d'un extrait de plante sont choisies parmi le guarana, la levure de riz rouge, le thé vert.

9. Composition selon l'une quelconque des revendications précédentes, dans laquelle les une ou plusieurs vitamines, enzymes et/ou co-enzymes sont choisies parmi la co-enzyme Q10, les vitamines C, D, A, B6, B12, D3, C et/ou la vitamine E.

10. Composition selon l'une quelconque des revendications précédentes, dans laquelle les une ou plusieurs parties d'une plante ou d'un extrait de plante, de préférence les parties d'un fruit ou légume ou d'un extrait de fruit ou légume, sont choisies parmi les framboises, les fraises, les mûres, les myrtilles, les griottes, les abricots, les pommes, les baies d'aronia, les groseilles rouges, les prunes, les citrons, les poires, les kiwis, les pêches, les bananes et/ou les ananas.

11. Composition selon l'une quelconque des revendications précédentes, dans laquelle la composition comprend des particules sous forme de vésicules comprenant de la vitamine E, et de l'huile d'olive, en plus d'au moins un minéral, oligoélément et/ou vitamine.

12. Composition selon l'une quelconque des revendications précédentes, dans laquelle la composition comprend :
a. au moins l'un parmi le manganèse, le calcium, le sélénium, le potassium,
b. au au moins l'un parmi la maca, la rhodiola, la schisandra, le probiotique,
c. au moins l'une parmi la coenzyme Q10, la vitamine C, D et
d. au moins l'un parmi les framboises, les fraises, les mûres, les myrtilles, les griottes, les abricots, les pommes, les baies d'aronia, les groseilles rouges, les prunes, les citrons, les poires, les kiwis, les pêches, les bananes et/ou les ananas,
ou dans laquelle la composition comprend :
a. au moins l'un parmi la cardamome, l'anis, les graines de lin, les probiotiques,
b. au moins l'une parmi la co-enzyme Q10, la vitamine A, B6, B12, D3, C, E,
c. au moins l'un parmi le fer, le zinc, le sélénium, le manganèse et
d. au moins l'un parmi les framboises, les fraises, les mûres, les myrtilles, les griottes, les abricots, les pommes, les baies d'aronia, les groseilles rouges, les prunes, les citrons, les poires, les kiwis, les pêches, les bananes et/ou les ananas.
ou dans laquelle la composition comprend :
a. au moins l'un parmi le guarana, la levure de riz rouge, le thé vert, le probiotique,
b. au moins l'une parmi la co-enzyme Q10, la vitamine C, B6, B12, D3,
c. au moins l'un parmi le zinc, le sélénium, le calcium, le manganèse et
d. au moins l'un parmi les framboises, les fraises, les mûres, les myrtilles, les griottes, les abricots, les pommes, les baies d'aronia, les groseilles rouges, les prunes, les citrons, les poires, les kiwis, les pêches, les bananes et/ou les ananas.

13. Procédé de fabrication d'une composition comme complément alimentaire et nutritionnel sous forme d'administration orale, comprenant :
(1) l'eau de mer,
(2) un ou plusieurs minéraux, oligo-éléments, vitamines, prébiotiques, probiotiques, enzymes, co-enzymes, parties d'une plante et/ou extraits de plantes, comprenant au moins une vitamine oléosoluble, et
(3) une ou plusieurs huiles,
dans lequel ledit procédé comprenant un procédé à base de nanoparticules comprenant l'homogénéisation et/ou la sonication d'un mélange d'eau de mer et d'huile, de sorte que la composition comprend des liposomes ou des vésicules de taille de nanoparticules, ou des mélanges de ceux-ci.

14. Procédé selon la revendication précédente, comprenant :
a. le mélange d'un ou de plusieurs éléments de composant (2) selon la revendication 14, comprenant une vitamine soluble dans l'huile, avec une ou plusieurs huile(s) ;
b. l'ajout du mélange d'huile de l'étape a. progressivement à l'eau de mer, éventuellement mélangée à des éléments supplémentaires de composant (2) selon la revendication 14, mélangeant ainsi le mélange d'huile de l'étape a. avec de l'eau de mer,
dans lequel l'ajout progressif se produit en ajoutant 10 à 35 % du mélange d'huile provenant de l'étape a. toutes les 10 à 15 minutes sur une période de 30 à 150 minutes, de préférence environ 45 à 75 minutes, de manière davantage préférée environ 60 minutes ;
c. le fait d'amener la température du mélange à l'étape b. à 40 à 60 degrés Celsius, de préférence environ 50 degrés Celsius ; pendant et/ou après le mélange à l'étape b.,
d. l'homogénéisation et/ou la sonication du mélange à l'étape b., pendant et/ou après le mélange à l'étape b. ; et
e. la formulation du mélange après l'étape d. à une composition sous forme de poudre lyophilisée, de comprimé, de gélule, de dragée, de comprimé sublingual, de granule, d'émulsion, de suspension, ou de composition adaptée à une administration orale.

15. Procédé selon l'une quelconque des revendications 13 ou 14, dans lequel la composition est préparée sous forme d'un mélange séché.
